# EUROPÄISCHE PATENTSCHRIFT

(11) **EP 1 879 534 B1**
(45) Veröffentlichungstag und Bekanntmachung des Hinweises auf die Patenterteilung: **08.06.2011**
(21) Anmeldenummer: 06724648.8
(22) Anmeldetag: 28.04.2006
(51) Int. Cl.: A61F 2/84

(54) **EINFÜHRSYSTEM MIT EINEM SELBST-EXPANDIERENDEN FLECHTSTENT**
INSERTION SYSTEM WITH A SELF-EXPANDING BRAIDED STENT
SYSTEME D'INTRODUCTION DOTE D'UNE ENDOPROTHESE VASCULAIRE AUTO-EXPANSIBLE

(30) Priorität: 04.05.2005 DE 102005020785
(43) Veröffentlichungstag der Anmeldung: 23.01.2008
(73) Patentinhaber: JOTEC GmbH, 72379 Hechingen (DE)
(72) Erfinder: KAUFMANN, Ralf, 72414 Rangendingen (DE); HAUSER, Berthold, 72393 Burladingen (DE)
(74) Vertreter: Laufer, Gabriele
(86) Internationale Anmeldenummer: PCT/EP2006/004026
(87) Internationale Veröffentlichungsnummer: WO 2006/117167

(56) Entgegenhaltungen:
- EP-A- 0 943 300
- US-A- 5 700 269
- US-A1- 2005 027 345
- US-B1- 6 350 278

## Beschreibung

Die vorliegende Erfindung betrifft ein Einführsystem mit einem selbstexpandierenden, zur Implantation in ein Blutgefäß vorgesehenen Flechtstent, mit einer ein distales und ein proximales Ende aufweisenden Außenhülle, in der eine relativ zu der Außenhülle verschiebbare Innenhülse angeordnet ist, die an dem proximalen Ende aus der Außenhülle mit einem Handhabungsabschnitt herausragt, und mit einer an dem distalen Ende angeordneten Spitze, die fest mit einem Stentträger verbunden ist, auf dem der Flechtstent in seinem geladenen Zustand angeordnet ist.

Ein derartiges Einführsystem ist der Anmelderin der vorliegenden Erfindung aus dem Markt bekannt.

Unter einem Stent versteht man eine radial expandierbare Endoprothese, die ein typisches intravaskuläres Implantat ist, das transluminal implantiert und radial vergrößert oder expandiert wird, nachdem es perkutan eingeführt wurde. Stents werden beispielsweise verwendet, um Blutgefäße zu verstärken und im vaskulären System eine Restenose nach einer vorhergehenden Angioplastie zu verhindern. Sie werden darüber hinaus auch zur Behandlung von Aneurysmen in Arterien implantiert.

Derartige Stents können selbstexpandierend sein oder durch eine von innen ausgeübte radiale Kraft expandiert werden, wenn sie beispielsweise auf einem Ballon montiert sind.

Je nach Anwendungsart werden die unterschiedlichsten Stents eingesetzt. Die hier vorliegende Anmeldung beschäftigt sich mit dem Applizieren von sogenannten Flechtstents, wie sie beispielsweise aus der DE 197 50 97 A1 oder der DE 103 35 649 A1 bekannt sind.

Ein Flechtstent ist ein Metallstent, der in sogenannter Leinwandbindungstechnik hergestellt wird. Er besteht aus einem in Längsrichtung streckbaren, hohlen Körper, dessen Mantel ein Geflecht aus einer Vielzahl von fadenförmigen Elementen ist, die im expandierten Zustand des Flechtstents eine Ebene senkrecht zur Längsrichtung unter einem Flechtwinkel schneiden. Ein Flechtstent erfährt bei seiner Streckung eine große Längenänderung, wobei die Längenänderung um so größer ist, je größer der ursprüngliche Durchmesser und je kleiner der ursprüngliche Flechtwinkel ist.

Zur Implantation wird ein derartiger Flechtstent langgestreckt in einem sogenannten Einführsystem oder Applikator gespeichert, das an geeigneter Stelle, bspw. der A. femoralis perkutan in den Körper eingeführt und transluminal bis zu dem Gefäß geführt wird, wo der Stent freigesetzt werden soll. An dem Einführsystem und an dem Stent sind dabei häufig Röntgenmarker vorgesehen, mit denen in situ die Positionierung und Freisetzung des Stents kontrolliert werden kann.

Bei Stents, die beim Freisetzen keine oder nur eine sehr geringe Längenveränderung erfahren, lässt sich auf diese Weise die Lage des zu implantierenden Stents problemlos überprüfen, weshalb es auch verschiedenste Einführsysteme für derartige, sich lediglich radial expandierende Stents gibt.

Bei den Flechtstents taucht jedoch das Problem auf, dass diese sich bei ihrer Freisetzung stark verkürzen. Bei einem Flechtwinkel von beispielsweise 40° beträgt das Verhältnis zwischen der Stentlänge im geladenen Zustand in dem Einführsystem und der Stentlänge im expandierten, also freien Zustand bspw. 1,5:1. Wenn der Flechtwinkel α kleiner wird, vergrößert sich dieses Verhältnis noch, bei einem Flechtwinkel von α = 10° kann das Verhältnis sogar 4:1 bis 6:1 sein. Es sei hier erwähnt, dass die Längenverkürzung natürlich auch vom Durchmesser des Stents im geladenen und vom Durchmesser des Stents im freigesetzten Zustand abhängt.

Flechtstents sind also extrem streckbar, wobei sie in ihrer langgestreckten Form sozusagen Masse speichern, die beim Zusammenziehen der Stents für einen dichten und steifen, funktionellen Bereich sorgt, wie es ausführlich in der eingangs erwähnten DE 103 35 649 A1 erwähnt ist.

Flechtstents, bei denen derartige Verkürzungen in Kauf genommen werden müssen, lassen sich bisher nur mit einer geringen Positioniergenauigkeit in dem Blutgefäß freisetzen. Die Positionierung eines Stents an der gewünschten Stelle in dem Blutgefäß ist nun jedoch ein kritischer Faktor, der die Wirkung der Stents und den Erfolg des medizinischen Eingriffs maßgeblich bestimmt. Der Bereich in dem Blutgefäß, in dem der Stent expandiert werden soll, ist üblicherweise für einen Mediziner nur schwer zugänglich.

Der Stent muss beispielsweise im Bereich eines Organes oder im Fall der A. Carotis im Bereich der Hirnarterien positioniert werden, wobei dort gegebenenfalls nur ein relativ kurzer Stent abgesetzt werden muss. Wegen der starken Längenverkürzung beim Freisetzen eines Flechtstents ist aber mit konventionellen Systemen dennoch eine große Freisetzungslänge erforderlich, wie es beispielsweise bei dem am Markt befindlichen System der Fall ist.

Der Flechtstent liegt bei dem bekannten Einführsystem im geladenen Zustand, also extrem langgestreckt, proximal von der Spitze auf dem Stentträger, der fest mit der Innenhülse verbunden ist. Der Stent wird dabei radial von der Außenhülle nach innen gedrückt und in seiner langgestreckten Form gehalten.

Zum Absetzen des Flechtstents wird die Innenhülse und damit der Stentträger sowie die mit diesem verbundene Spitze festgehalten, sie darf während der Freisetzung nicht bewegt werden. Daraufhin wird die Außenhülle vorsichtig von der Spitze zurückgezogen, so dass der Stent allmählich freigesetzt wird. Der freikommende Stent zieht sich dabei in Richtung der Außenhülle zusammen, entfernt sich also von der Spitze, die durch die Innenhülse gegenüber dem Gefäß feststehend positioniert bleibt. Beim Zurückziehen der Außenhülle folgt also der allmählich freigesetzte und sich radial expandierende, aber in Längsrichtung kontrahierende Stent zunächst der Außenhülle, bis ein derartiger Abschnitt des Flechtstents freigesetzt wurde, dass dieser sich fest gegen die Innenwand des Gefäßes legt.

Aus all dem ergibt sich jedoch, dass die Spitze des Einführsystems deutlich über die vorgesehene Absetzstelle des Flechtstents distal vorgeschoben werden muss, um eine richtige Platzierung zu ermöglichen. Bei dem bekannten Einführsystem sind dies mehrere Zentimeter, was zu einer Freisetzungslänge führt, die insbesondere bei kleinen Flechtwinkeln nicht tolerierbar ist. Insbesondere dann, wenn ein relativ kurzer Stent in der Nähe eines Organes oder der Hirnarterien positioniert werden muss, besteht dabei nämlich die Gefahr, dass durch die weiter vorgeschobene Spitze Beschädigungen des Organes oder der Hirnarterie erfolgen.

Ein weiterer Nachteil bei dem bekannten Einführsystem ist darin zu sehen, dass die Lage des Flechtstents in dem Gefäß nur schwer durch Manipulationen an der Außenhülle beeinflusst werden kann. Wenn der Operateur das Einführsystem zu weit oder nicht weit genug eingeschoben hat, kann er die endgültige Lage des Flechtstents bei dem bekannten Einführsystem nicht mehr beeinflussen. Wenn er feststellt, dass der Flechtstent zu weit oberhalb oder unterhalb von der geplanten Absetzstelle freigesetzt wird, muss er den Flechtstent wieder einziehen, was bei dem bekannten Einführsystem jedoch nur in Notfällen und nicht mehr als zweimal zulässig ist. Zum "Rückladen" des Flechtstents in das Einführsystem wird jetzt die Außenhülle festgehalten, während die Innenhülse vorsichtig zurückgezogen wird. Dieses Rückladen ist bei dem bekannten Einführsystem jedoch nur möglich, solange noch nicht mehr als 50% des Flechtstents freigesetzt wurde.

Bei dem bekannten Einführsystem ist also zum einen von Nachteil, dass damit insbesondere Flechtstents mit kleinen Flechtwinkeln nicht nahe genug an Organe oder Hirnarterien etc. heran positioniert werden können, während darüber hinaus weiter von Nachteil ist, dass die genaue Positionierung extrem schwierig ist.

Die EP 0 943 300 A1 offenbart ein Einführsystem für Endoprothesen, mit einer Innenhülse, die von einer Außen. Bzw. Rückzugshülle umgeben ist, wobei die Innenhülse von außen gesteuerte Rückhaltemittel aufweist, bspw. einen aufblasbaren Ballon, nach dessen Inflatierung die Endoprothese relativ zu der Innenhülse fixiert und durch dessen Deflation freigesetzt werden kann. Durch diese Rückhaltemittel soll es möglich sein, die Endoprothese genauer zu positionieren.

Ferner offenbart die US 5,700,269 ein Einführsystem für Endoprothesen, mit einem Kolben, der zur Ladung eines Stents verschieblich auf einem Stentträger angeordnet ist. Das Einführsystem weist ferner eine Außenhülle auf, über deren Rückzug und bei gleichzeitigem Gegenhalten des Kolbens der Stent freigesetzt werden kann. Das hierin offenbarten Einführsystem weist ferner Verschlussmechanismen auf, mit welchen die Innenhülse gegenüber dem Stentträger zur Freisetzung des Stents fixiert werden kann, sowie einen Mechanismus, mit dem die Innenhülse gegenüber der Außenhülle zur Ladung des Stents in das System fixiert werden kann.

In der US 6,350,278 ist ein Einführsystem für eine Prothese offenbart, mit einem Stentträger und einer den Stent komprimierenden Außenhülle. Das Einführsystem kann ferner noch eine weitere Hülle aufweisen, die die Kombination aus Stentträger und Außenhülle aufnimmt.

Schließlich offenbart die US 2005/0027345 ein Einführsystem für einen Stent mit einer Rückzugshülle und einem mit einem Pusher fest verbundenen Stentträger.

Vor diesem Hintergrund liegt der vorliegenden Erfindung die Aufgabe zugrunde, das bekannte Einführsystem derart weiterzuentwickeln, dass auf konstruktiv einfache Weise Flechtstents mit verbesserter Positioniergenauigkeit freigesetzt werden können, wobei die Freisetzungslänge vorzugsweise verringert werden soll.

Diese Aufgabe wird erfindungsgemäß auch Einführsystem gemäß Anspruch 1 gelöst.

Die der Erfindung zugrunde liegende Aufgabe wird auf diese Weise vollkommen gelöst.

Die Erfinder der vorliegenden Anmeldung haben nämlich erkannt, dass die Probleme bei dem bekannten Einführsystem u.a. darauf zurückzuführen sind, dass der Stentträger Teil der Innenhülse ist. Erfindungsgemäß wird der Stentträger nun jedoch von der Innenhülse entkoppelt, so dass der Flechtstent entweder durch Zurückziehen der Außenhülle oder durch Vorschieben der Innenhülse freigesetzt werden kann. Im Gegensatz zu dem bekannten Flechtsystem lässt sich jetzt nämlich die Innenhülse relativ zu dem Stentträger verschieben.

Der Stent wird also erfindungsgemäß durch die Relativbewegung zwischen Außenhülle und Innenhülse freigesetzt, der Operateur kann damit auch an der Außenhülle manipulieren und die Lage des Stents in situ verändern, wenn er bei der Freisetzung bemerkt, dass er das Einführsystem noch nicht weit genug bzw. bereits zu weit eingeschoben hat.

Zu Beginn der Freisetzung des Flechtstents kommt durch die Relativbewegung zwischen Außenhülle und Innenhülse zunächst die Spitze von der Außenhülle frei, wobei auch das distale Ende des Stents aus der Außenhülle herausgelangt und sich expandiert, also von der Spitze freikommt. Durch Vorschieben der Innenhülse wird jetzt der Stent von dem Stentträger heruntergeschoben, so dass sich der Stent über die Spitze schieben kann, da diese nicht mehr an die Innenhülse gekoppelt ist. Beim Freisetzen kann es dabei angezeigt sein, zunächst die Außenhülle etwas zurückzuziehen, bis der Stent von der Spitze freikommt, und dann die Innenhülse vorzuschieben. Durch abwechselndes Arbeiten mit Innenhülse und Außenhülle oder durch gemeinsames Verschieben von Innenhülse und Außenhülle kann der Stent jetzt optimal positioniert werden. Die starken Verkürzungen bei der Freisetzung führen zwar auch hier zu nicht genau vorhersagbaren Verlagerungen des Stents, diese können jedoch durch Relativbewegungen in beide Richtungen ausgeglichen werden.

Da die Spitze zudem nur geringfügig oder gar nicht über den gewünschten Freisetzungsort distal vorsteht, besteht auch keine Gefahr, distal der Absetzstelle gelegene Gefäße oder Organe zu verletzen.

Darüber hinaus kann der Stent durch diese neue Handhabungsmöglichkeit richtiggehend "aufgebaut" werden, er kann also an verschiedenen Stellen unterschiedlich dicht gelegt werden, um beispielsweise insbesondere im Bereich eines Aneurysma eine sehr dichte Metallstruktur zu erzeugen, während er vor und hinter dem Aneurysma etwas gestreckter gelegt wird, damit er sich fest gegen die Innenwände des Gefäßes verankern kann.

Durch die Entkopplung der Spitze und des Stentträgers von der Innenhülse ermöglicht das neue Einführsystem also zusammengefasst die Positionierung sich bei der Freisetzung extrem verkürzender Flechtstents auch an bisher nicht zugänglichen Gefäßstellen, wobei das neue Einführsystem die Eigenschaften der Flechtstents optimal zur Geltung bringt, indem nämlich die Flechtstents bei der Freisetzung noch entsprechend aufgebaut oder gestreckt werden können.

Ferner ist zwischen Außenhülle, und Stentträger ein Verriegelungsmechanismus vorgesehen, der die Bewegung des Stentträgers relativ zu der Außenhülle in distaler Richtung auf einen maximalen Verfahrweg begrenzt.

Bei dieser Maßnahme ist von Vorteil, dass die Spitze beim anfänglichen Freisetzen des Flechtstents nicht zu weit vorgeschoben wird. Ferner ist von Vorteil, dass beim weiteren Freisetzen des Flechtstents durch Zurückziehen der Außenhülle die Spitze mit zurückgezogen wird, also in proximaler Richtung in den sich expandierenden Stent hinein. Ferner wird durch diese Maßnahme erreicht, dass beim Vorschieben der Innenhülse relativ zu der Außenhülle der Stentträger und damit die Spitze stehen bleiben, so dass sichergestellt ist, dass der Flechtstent sozusagen über die Spitze hinübergeschoben werden kann.

Diese Maßnahme ermöglicht es damit, den Flechtstent in distaler Richtung vor der ursprünglichen Lage der Spitze abzusetzen. Das Einführsystem wird dazu so weit in das Gefäß hineingeschoben, bis sich die Spitze kurz vor dem Punkt befindet, an dem der freigesetzte Stent distal enden soll. Jetzt wird die Außenhülle ein kleines Stück zurückgezogen, so dass die Spitze und der Flechtstent an seinem distalen Ende freikommen. Dann wird die Innenhülse vorgeschoben, wodurch sich der Stent über die Spitze schiebt, so dass er mit seinem distalen Ende distal von der Spitze in Anlage mit der Innenwand des Blutgefäßes gelangt. In bestimmten Fällen kann diese Reihenfolge auch umgekehrt werden, so dass zunächst die Innenhülse vorgeschoben wird, bis der Flechtstent am Blutgefäß anliegt, woraufhin dann die Außenhülle zurückgezogen wird, bzw. eine weitere Relativbewegung zwischen Außenhülle und Innenhülse erfolgt.

Durch weitere Relativbewegungen zwischen Außenhülle und Innenhülse kann der Flechtstent jetzt entsprechend aufgebaut und abgesetzt werden. Vom Ergebnis her bedeutet dies, dass die Spitze nicht in Bereiche des Gefäßes vorgeschoben werden muss, in denen der Stent nach endgültiger Freisetzung gar nicht zu liegen kommt. Dies ist ein entscheidender Vorteil gegenüber bekannten Freisetzungssystemen.

Dabei ist es weiter bevorzugt, wenn der Verriegelungsmechanismus die Bewegung des Stentträgers relativ zu der Außenhülle in proximaler Richtung dann begrenzt, wenn der Stentträger zuvor relativ zu der Außenhülle um einen vorgegebenen Verfahrweg in distaler Richtung verfahren wurde.

Bei dieser Maßnahme ist von Vorteil, dass ein vollständiges Zurückladen auch des fast vollständig freigesetzten Flechtstents möglich ist. Beim Zurückziehen des zu diesem Zweck geeignet mit der Innenhülse verbundenen Stents wird die Spitze nämlich nicht mitgenommen, sondern durch den Verriegelungsmechanismus gegenüber der Außenhülle festgehalten, so dass wieder die relative Bewegung zwischen Stentträger und Innenhülse möglich ist. Dadurch wird sozusagen der Flechtstent wieder auf den Stentträger aufgezogen, wobei durch die Arretierung der Spitze verhindert wird, dass sich der Flechtstent zwischen Außenhülle und Spitze verklemmt.

Zwar wäre es auch möglich, die relative Bewegung zwischen Stentträger und Innenhülse durch unterschiedliche Reibungskräfte oder durch Federn zu ermöglichen, es hat sich jedoch herausgestellt, dass bei den geringen Abmaßen und Durchmessern, die bei dem neuen Einführsystem vorhanden sind, eine rein mechanische Arretierung die bessere Lösung darstellt.

Bei dem neuen Einführsystem sind also Spitze und Stentträger sowohl relativ zu der Außenhülle als auch relativ zu der Innenhülse frei beweglich, wobei entsprechende Maßnahmen vorgesehen sein können, um die Verschiebbarkeit des Stentträgers zu der Außenhülle sowohl in distale Richtung als auch in proximale Richtung zu begrenzen. Obwohl diese Begrenzung der Bewegungsfreiheit durch Federkräfte oder Reibungskräfte bewirkt werden kann, werden gemäß einer bevorzugten Ausführungsform der Erfindung hierzu Verriegelungsmechanismen verwendet.

Dabei ist es weiter bevorzugt, wenn mit der Innenhülse ein Entriegelungsmechanismus verbunden ist, der bei proximaler Bewegung der Innenhülse relativ zu der Außenhülle die relative Bewegung des Stentträgers zu der Außenhülle wieder freigibt.

Hier ist von Vorteil, dass die Verriegelung der Bewegung des Stentträgers relativ zu der Außenhülle in proximaler Richtung schließlich wieder aufgehoben wird, so dass die Spitze dann doch noch wieder bis an die Außenhülle zurückgezogen werden kann, wenn zuvor der Stent vollständig wieder auf den Stentträger aufgezogen wurde. Dies ermöglicht eine erneute Positionierung des gesamten Einführsystems in distaler oder proximaler Richtung sowie ein einfaches vollständiges Entfernen des bereits teilweise freigesetzten Stents, wenn während der Operation entsprechende Indikationen auftreten.

Darüber hinaus ist es auf diese Weise möglich, nach dem vollständigen Freisetzen des Stents die Spitze in proximaler Richtung in die Außenhülle zurückzuziehen, bevor das Einführsystem endgültig aus dem Körper zurückgezogen wird.

In diesem Zusammenhang ist es bevorzugt, wenn der Stentträger an seinem proximalen Ende mit einer Steuerstange verbunden ist, die die Innenhülse zentrisch durchsetzt und an dem proximalen Ende aus der Innenhülle herausragt.

Hier ist von Vorteil, dass die Steuerstange es ermöglicht, den Stentträger und damit die Spitze aktiv translatorisch gegenüber der Innenhülse zu verfahren und festzuhalten, so dass auf Verriegelungs- und Entriegelungsmechanismus verzichtet werden kann. Der Verriegelungsmechanismus und gegebenenfalls der Entriegelungsmechanismus können - falls gewünscht - dabei auch im Bereich des proximalen Endes vorgesehen sein, so dass sie außerhalb des Körpers bleiben. Allerdings ist zu bedenken, dass der Operateur neben der Außenhülle und der Innenhülse bei dieser Konstruktion auch die Steuerstange manipulieren muss.

Alternativ ist es daher bevorzugt, wenn der Stentträger an seinem proximalen Ende in der Innenhülse gelagert ist, die einen Längsschlitz aufweist, durch den ein mit der Außenhülle verbundener erster Anschlag in die Innenhülse hineinragt, der die Bewegung des Stentträgers relativ zu der Außenhülle in distaler Richtung auf den maximalen Verfahrweg begrenzt.

Bei dieser Maßnahme ist von Vorteil, dass die frei bewegliche Spitze sowie der damit verbundene Stentträger sozusagen ohne eigenen Antrieb ausgestaltet sind, wobei der Anschlag nur in distale Richtung wirkt. Der maximale Verfahrweg, um den also die Spitze gegenüber der Außenhülle in distale Richtung bewegt werden kann, wird dabei durch den axialen Abstand zwischen diesem ersten Anschlag und einer Gegenfläche an dem Stentträger bestimmt, wenn der Stent noch in geladenem Zustand ist.

Dabei ist es bevorzugt, wenn der Stentträger fest mit einem verschieblich in der Innenhülse gelagerten distalen Steuerblock verbunden ist, der auf Zug mit einem verschieblich in der Innenhülse gelagerten proximalen Steuerblock verbunden ist, der bei relativer Bewegung des Stentträgers zu der Außenhülle in distaler Richtung um den maximalen Verfahrweg mit dem ersten Anschlag in Anlage gelangt.

Diese Maßnahme ist konstruktiv von Vorteil, der in distale Richtung wirkende Verriegelungsmechanismus ist einfach aufgebaut, in dem sowieso vorhandenen Lumen der Innenhülse lassen sich auf mikromechanische Weise die beiden Steuerblöcke problemlos installieren.

Insgesamt ist es dabei bevorzugt, wenn dem Längsschlitz ein mit dem Stentträger verbundener weiterer Anschlag zugeordnet ist, der in Anlage mit einer Ausnehmung der Außenhülle gelangt und die Bewegung des Stentträgers relativ zu der Außenhülle in proximaler Richtung begrenzt, wenn der Stentträger zuvor relativ zu der Außenhülle um den vorgegebenen Verfahrweg in distaler Richtung verfahren wurde.

Auch diese Maßnahme ist konstruktiv von Vorteil. Der weitere Anschlag wird zunächst in der Innenhülse gehalten, erst wenn die Innenhülse relativ zu dem Stentträger weiter distal verschoben wurde, kommt der weitere Anschlag frei und kann durch den Längsschlitz in der Innenhülse hindurch in die Außenhülle eingreifen, wodurch verhindert wird, dass beim Zurückziehen des Stents durch Zurückziehen der Innenhülse durch die Reibung zwischen Stentträger und Stent die Spitze in proximale Richtung in die Außenhülle hineingezogen wird.

Dabei ist es bevorzugt, wenn der weitere Anschlag an dem distalen Steuerblock angeordnet ist.

Hier ist von Vorteil, dass der Längsschlitz in der Innenhülse zum Entriegeln des weiteren Anschlages dienen kann. Wenn die Innenhülse nämlich zurückgezogen wird, drückt der in der Innenhülse vorgesehene Längsschlitz mit seinem distalen Ende den weiteren Anschlag nach innen, so dass er aus der Außenhülle freikommt und die Spitze jetzt in die Außenhülle eingezogen wird. Weil der weitere Anschlag an dem distalen Steuerblock angeordnet ist, folgt die Entriegelung auch automatisch erst dann, wenn die Innenhülse fast vollständig wieder in ihre ursprüngliche Position zurückgezogen wurde, der Flechtstent also sicher wieder auf dem Stentträger aufgezogen wurde.

Allgemein ist es dabei bevorzugt, wenn der distale und der proximale Steuerblock über einen Verbindungsdraht miteinander verbunden sind, der vorzugsweise durch ein in dem ersten Anschlag vorgesehenes Führungsloch verläuft.

Auch bei dieser Maßnahme ist die einfache Konstruktion von Vorteil, es sind lediglich zwei Steuerblöcke in der Innenhülse anzuordnen, wobei sich der Verbindungsdraht wegen seiner Führung durch das Führungsloch auch nicht verknicken oder verknoten kann, wenn die Spitze schließlich zurückgezogen wird. Andererseits ist es bevorzugt, wenn der distale und der proximale Steuerblock an gegenüber liegenden Enden einer in der Innenhülse verschieblich gelagerten Steuerhülse ausgebildet sind, in der ein Längsschlitz vorgesehen ist, in den der erste Anschlag eingreift.

Dies ist eine Alternative zu der Lösung mit dem Verbindungsdraht, die konstruktiv ebenfalls einfach aufgebaut ist. Ein weiterer Vorteil besteht hier darin, dass auch der proximale Steuerblock beim Zurückziehen der Innenhülse mitbewegt wird.

Bei vielen Ausführungsformen der Erfindung ist es dabei bevorzugt, wenn auf den Stentträger ein längs verschiebbarer Gleitkörper angeordnet ist, der bei geladenem Flechtstent zwischen Flechtstent und Spitze liegt.

Bei dieser Maßnahme ist von Vorteil, dass überraschenderweise ein Verklemmen des Flechtstents zwischen Außenhülle und Spitze bzw. Gleitkörper verhindert wird, wenn der Flechtstent wieder zurückgeladen wird. Der Gleitkörper stellt damit eine alternative oder ergänzende Möglichkeit zu dem Verriegelungsmechanismus dar, der die Bewegung des Stentträgers relativ zu der Außenhülle in proximaler Richtung dann begrenzt, wenn der Stentträger zuvor relativ zu der Außenhülle um einen vorgegebenen Verfahrweg in distaler Richtung verfahren wurde.

Die Erfinder der vorliegenden Anmeldung haben nämlich erkannt, dass dieser Gleitkörper beim Zurückziehen des Stents auf den Stentträger eine Art oszillierende Rüttelbewegung zwischen Spitze und Flechtstent durchführt, er wird kurzfristig eingeklemmt und springt dann in Richtung Spitze zurück, woraufhin der Flechtstent dann weiter eingezogen werden kann. Auch hierdurch wird erreicht, dass ein fast vollständig freigesetzter Flechtstent wieder rückgeladen werden kann.

Dabei ist es bevorzugt, wenn der Gleitkörper einen Außendurchmesser aufweist, der größer ist als die Differenz zwischen dem Innendurchmesser der Außenhülle an deren distalem Ende und der doppelten Wandstärke des Flechtstents.

Diese Maßnahme ist konstruktiv von Vorteil, denn sie sorgt dafür, dass der Flechtstent nicht so einfach zwischen Gleitkörper und Außenhülle verklemmen kann.

Weiter ist es bevorzugt, wenn der Gleitkörper an seinem pröximalen Ende abgerundet ist und vorzugsweise aus einem Material gefertigt ist, das gegenüber der Innenwand des Flechtstents einen geringen Gleitreibungskoeffizienten aufweist, wobei der Gleitkörper weiter vorzugsweise aus einem elastischen Material gefertigt ist.

Auch diese Maßnahmen führen einzeln und in Kombination dazu, dass der Flechtstent nicht so leicht zwischen Gleitkörper und distalem Ende der Außenhülle verklemmt, wobei ein aus elastischem Material gefertigter Gleitkörper nach einem möglichen Einklemmen zurückspringt, da er die gespeicherte elastische Deformationsenergie fast vollständig in kinetische Energie einwandelt und axial in distale Richtung zurückspringt.

Weiter ist es dabei bevorzugt, wenn das distale Ende der Außenhülle versteift ist.

Dies kann beispielsweise durch Röntgenmarker erfolgen, die das distale Ende der Außenhülle verstärken, so dass diese sich nicht aufweiten kann. Auch dies führt dazu, dass der Flechtstent sich beim Zurückziehen nicht zwischen Gleitkörper und distalem Ende der Außenhülle verklemmen kann.

Allgemein ist es noch bevorzugt, wenn auf dem Handhabungsabschnitt der Innenhülse ein Distanzstück fixiert ist, das ein Einschieben der Innenhülse in die Außenhülle begrenzt.

Diese Maßnahme ist konstruktiv von Vorteil, sie stellt auf einfache Weise ein Sicherheitselement bei der Bedienung des neuen Einführsystems bereit. Das Distanzstück ist nämlich so angebracht, dass es anzeigt, wann der Stent kurz vor der endgültigen Freisetzung steht. Es muss entfernt werden, bevor die Freisetzung abgeschlossen werden kann. Solange der Operateur also dieses Distanzstück nicht entfernt, kann er bei seinen Manipulationen den Flechtstent nicht so weit freisetzen, dass er nicht mehr zurückgezogen werden kann.

Gemäß Anspruch 1 weist ferner, die Innenhülse an ihrem distalen Ende eine Halterung auf, über die der Flechtstent an seinem proximalen Ende auf Zug mit der Innenhülse verbunden ist.

Diese Maßnahme hat zum Vorteil, dass ein problemloses Rückladen des Stents ermöglicht wird, der durch Rückziehen der Innenhülse wieder auf dem Stentträger aufgezogen werden kann.

Dabei ist es bevorzugt, wenn die Halterung einen Flansch aufweist, auf dem das proximale Ende des Flechtstents durch die Außenhülle fixiert wird.

Auch diese Maßnahme ist konstruktiv von Vorteil, der Flansch stellt eine Art Widerhaken dar, der sich von selbst löst, wenn die Innenhülse so weit in die Außenhülle hineingeschoben wird, dass der Flansch aus dem distalen Ende der Außenhülle herausragt. Hierzu ist es gegebenenfalls erforderlich, vorher das Distanzstück von der Innenhülse zu entfernen.

Ein weiterer Vorteil bei dieser Konstruktion besteht darin, dass das proximale Ende des Stents so nicht beschädigt wird, wie es bei einer Haltevorrichtung der Fall sein könnte, aus der der Flechtstent herausgezogen werden muss.

Allgemein ist es noch bevorzugt, wenn zentral durch die Spitze und den Stentträger ein Kanal für einen Führungsdraht verläuft, der seitlich durch Längsschlitze aus der Innenhülse und der Außenhülle herausgeführt ist.

Diese Maßnahme ist an sich aus dem Stand der Technik bekannt, der Führungsdraht wird vor dem eigentlichen Einführen des Einführsystems gelegt, um die Spitze des Einführsystems sicher durch die Windungen der Gefäße hindurchleiten zu können. Dieses Verfahren ist allgemein als Seldinger-Methode bekannt.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachstehenden Beschreibung näher erläutert. Es zeigen:
- Fig. 1: in schematischer Seitenansicht ein Einführsystem für einen Flechtstent;
- Fig. 2: einen vergrößerten, schematischen und nicht maßstabs- getreuen Längsschnitt durch den distalen Abschnitt des Einführsystems aus Fig. 1 in einem ersten Ausfüh- rungsbeispiel;
- Fig. 3: in einer Darstellung wie Fig. 2 ein zweites Ausfüh- rungsbeispiel des neuen Einführsystems;
- Fig. 4: in einer Darstellung wie Fig. 2, jedoch mit teilweise freigesetztem Flechtstent, ein drittes Ausführungs- beispiel des neuen Einführsystems;
- Fig. 5: eine Darstellung wie Fig. 4, jedoch mit teilweise wieder in die Außenhülle hineingezogenem Flechtstent;
- Fig. 6: eine Darstellung wie Fig. 4, jedoch mit völlig frei- gesetztem Flechtstent;
- Fig. 7: in einer Darstellung wie Fig. 2 ein viertes Ausfüh- rungsbeispiel des neuen Einführsystems;
- Fig. 8: eine Darstellung wie Fig. 7, jedoch mit teilweise freigesetztem Flechtstent; und
- Fig. 9: eine Darstellung wie Fig. 8, jedoch mit vollständig freigesetztem Flechtstent.

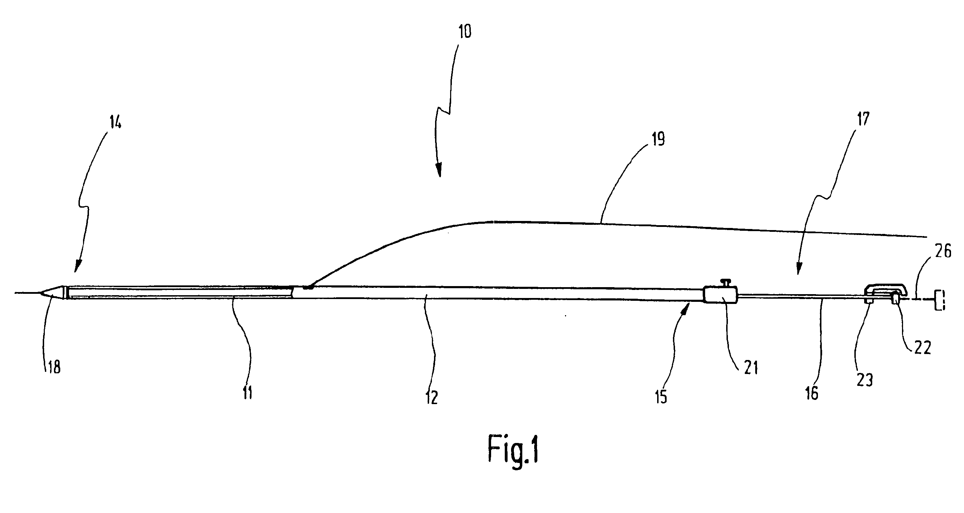

In Fig. 1 ist schematisch mit 10 ein Einführsystem bezeichnet, mit dem ein bei 11 angedeuteter Flechtstent in ein Blutgefäß implantiert werden kann.

Der Flechtstent 11 ist ein selbstexpandierender Metallstent, der in Leinwandbindungstechnik hergestellt ist, wie dies in der eingangs erwähnten DE 103 35 649 beschrieben ist. Aufgrund seines Flechtwinkels von α = 10° weist der Flechtstent 11 in dem in Fig. 1 gezeigten, in das Einführsystem 10 geladenen Zustand eine Länge auf, die ca. fünf mal länger ist als die Länge des Flechtstents im freigesetzten Zustand.

Das Einführsystem 10 weist eine Außenhülle 12 auf, an derem distalen Ende 14 der Flechtstent 11 angeordnet ist, sowie ein gegenüberliegendes proximales Ende 15.

Gemäß üblicher Nomenklatur wird mit distaler Richtung eine auf den Patienten weisende Richtung bezeichnet, in den der Flechtstent 11 implantiert werden soll. Mit proximaler Richtung wird demgemäß eine auf den Operateur weisende Richtung bezeichnet.

In der Außenhülle 12 ist gegenüber dieser verschiebbar eine Innenhülse 16 angeordnet, die an dem proximalen Ende 15 der Außenhülle 12 aus dieser herausragt und dort einen Handhabungsabschnitt 17 aufweist.

An dem distalen Ende 14 weist das Einführsystem 10 eine Spitze 18 auf, durch die ein mit 19 bezeichneter Führungsdraht verläuft, der seitlich aus der Außenhülle 12 herausragt. Mit diesem Führungsdraht wird gemäß der an sich bekannten Seldinger-Methode das Einführsystem 10 in ein Blutgefäß eines Patienten eingeführt, um dort den Flechtstent 11 freizusetzen.

Für die dabei erforderlichen Manipulationen ist an dem proximalen Ende 15 der Außenhülle 12 ein Zug-Griff 21 mit Spülanschluss angeordnet, wie er allgemein bei derartigen Einführsystemen bekannt ist. An dem Handhabungsabschnitt 17 der Innenhülse 16 ist ebenfalls ein Handgriff 22 angeordnet, über den die Innenhülse 16 relativ zu der Außenhülle 12 verschoben werden kann. In distaler Richtung vor dem Handgriff 22 ist noch ein Distanzstück 23 angedeutet, das als Anschlag beim Einschieben der Innenhülse 16 in die Außenhülle dient und mit dem Zug-Griff 21 in Anlage gelangt.

Das Distanzstück 23 verhindert es, dass die Innenhülse 16 so weit in die Außenhülle 12 hineingeschoben wird, dass der Flechtstent vollständig freigesetzt wird. Er stellt also ein Sicherheitselement dar, durch das vorzeitiges Freisetzen des Flechtstents 11 verhindert wird. Wenn das Distanzstück 13 entfernt wird, lässt sich die Innenhülse 16 weiter in die Außenhülle 12 hineinschieben, um den Flechtstent 11 endgültig freizusetzen.

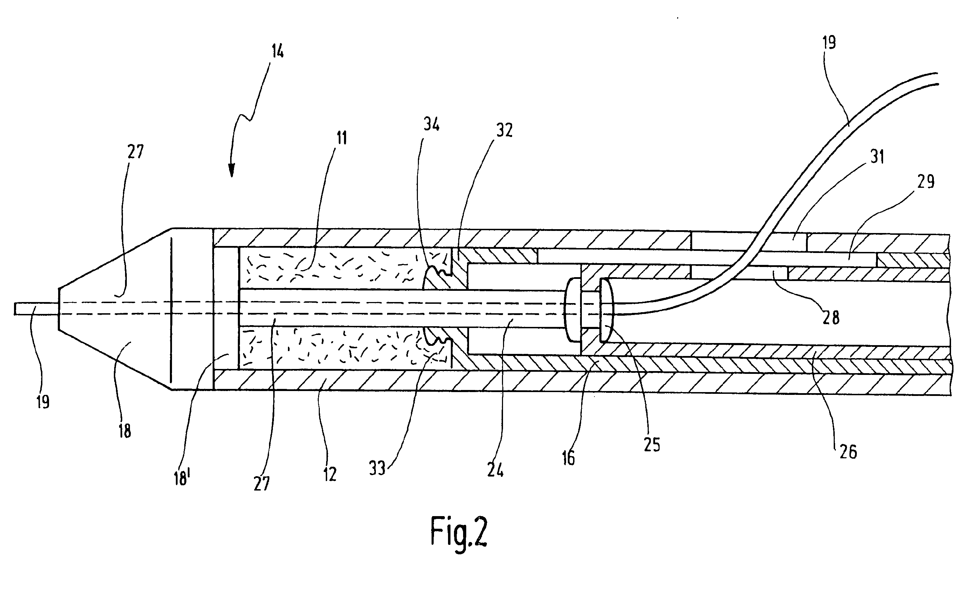

In Fig. 2 ist in schematischem Längsschnitt das Einführsystem 10 aus Fig. 1 in vergrößerter, nicht maßstabsgetreuer und schematischer Darstellung gezeigt.

Die Spitze 18 sitzt mit ihrem Flansch 18' innen in der Außenhülle 12, so dass das Innere der Außenhülse 12 nach außen verschlossen ist. An die Spitze 18 schließt sich in Fig. 2 in proximaler Richtung ein fest mit der Spitze 18 verbundener Stentträger 24 an, der in die Innenhülse 16 hineinragt und dort an seinem proximalen Ende 25 fest mit einer Steuerstange 26 verbunden ist, die die Innenhülse 16 zentrisch durchsetzt und an dem Handgriff 22 aus der Innenhülse 16 herausragt, wie es in Fig. 1 gestrichelt dargestellt ist.

Sowohl die Spitze 18 als auch der Stentträger 24 sind von einem Kanal 27 durchsetzt, durch den der Führungsdraht 19 verläuft, der durch Längsschlitze 28, 29 und 31 in der Steuerstange 26, der Innenhülse 16 bzw. der Außenhülle 31 hindurch nach außen verläuft. Die Längsschlitze 28, 29 und 31 sind dabei so gewählt, dass der Führungsdraht 19 beim Verschieben der Innenhülse 16 relativ zu der Außenhülle 12 und beim Verschieben des Stentträgers 24 über die Steuerstange 26 nicht verknickt oder verklemmt.

Die Innenhülse 16 weist an ihrem distalen Ende 32 eine Halterung auf, über die der Flechtstent 11 an seinem proximalen Ende 33 auf Zug mit der Innenhülse 16 verbunden ist. Hierzu ist an dem distalen Ende 32 der Innenhülse 16 ein Flansch 34 vorgesehen, auf dem das proximale Ende 33 des Flechtstents 11 aufsitzt und dort durch radial nach innen gerichteten Druck der Außenhülle 12 gehalten wird.

Die Konstruktion ist in Fig. 2 jetzt so getroffen, dass Außenhülle 12, Innenhülse 16 und Stentträger 24 gegeneinander verschiebbar sind. Zum Freisetzen des Flechtstents 11 wird beispielsweise zunächst die Außenhülle 12 zurückgezogen, wozu die Innenhülse 16 und die Steuerstange 26 festgehalten werden. Auf diese Weise kommt die Spitze 18 aus der Außenhülle 12 frei, so dass sich der Flechtstent 11 radial nach außen erweitern kann, wobei er sich gleichzeitig verkürzt. Alternativ kann auch zunächst die Innenhülse 16 relativ zu der Außenhülse 12 vorgeschoben werden, bis der Flechtstent 11 sich an das Blutgefäß anlegt. Daraufhin wird die Außenhülle 12 zurückgezogen, um den Flechtstent 11 weiter freizusetzen.

Zum weiteren Freisetzen des Flechtstents 11 kann dann die Innenhülse 16 relativ zu der Außenhülle 12 verschoben werden, wodurch der Flechtstent 11 von dem Stentträger 24 heruntergeschoben wird. Damit durch die dabei entstehende Reibung zwischen dem Flechtstent 11 und dem Stentträger 24 die Spitze 18 nicht weiter in distale Richtung geschoben wird, wird die Steuerstange 26 zusammen mit der Außenhülle 12 unverschieblich gehalten. Dies kann entweder durch den Operateur erfolgen, wobei es auch möglich ist, Steuerstange 26 und Außenhülle 12 am proximalen Ende 15 bedarfsweise miteinander zu verriegeln.

Wenn die Freisetzung des Flechtstents 11 nicht zufriedenstellend verläuft, kann der Flechtstent 11 auch wieder auf den Stentträger 24 aufgezogen werden, indem nämlich die Innenhülse 16 wieder in die Außenhülle 12 hineingezogen wird. Damit dabei die Spitze 18 sich nicht gegen den wieder in die Außenhülle 12 eingezogenen Stent verklemmt, wird die Spitze 18 jetzt über die Steuerstange 26 soweit vor die Außenhülle 12 geschoben und gehalten, dass der Flechtstent 11 problemlos wieder in die Außenhülle 12 und auf den Stentträger 24 gezogen werden kann.

Auf diese Weise ist es sogar möglich, den Flechtstent 11 wieder vollständig in das Einführsystem 10 zurückzuladen, und danach die Spitze 18 wieder einzuziehen.

Das Einführsystem 10 kann dann bei bestimmter Indikation vollständig wieder entnommen werden, wobei es auch möglich ist, die Position des Einführsystems in dem Blutgefäß lediglich zu korrigieren und den Flechtstent 11 dann erneut freizusetzen.

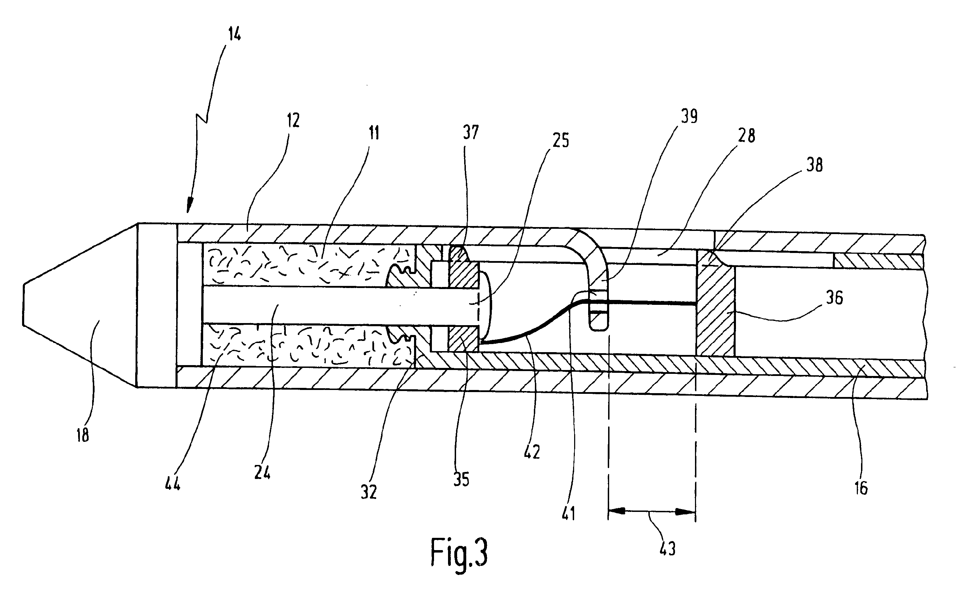

In Fig. 3 ist in einer Darstellung wie Fig. 2 - jedoch ohne Darstellung des Führungsdrahtes 19 - ein zweites Ausführungsbeispiel des neuen Einführsystems gezeigt, bei dem auf eine aktive Steuerung der translatorischen Bewegung der Spitze 18 und damit des Stentträgers 24 verzichtet wird, vielmehr ist die Spitze 18 hier frei gegenüber der Außenhülle 12 sowie der Innenhülse 16 beweglich angeordnet. Zu diesem Zweck ist der Stentträger 24 an seinem distalen Ende in der Innenhülse 16 an einem distalen Steuerblock 35 gelagert, dem ein proximaler Steuerblock 36 zugeordnet ist. Der distale Steuerblock 35 und der proximale Steuerblock 36 sind rotationssymmetrisch ausgeführt, weisen jedoch eine Nase 37 bzw. 38 auf, mit der sie in dem Längsschlitz 28 der Innenhülse 16 geführt sind, so dass sie nicht verkanten können.

Durch den Längsschlitz 28 ragt ein einstückig mit der Außenhülle 12 ausgebildeter Anschlag 39 hinein, in dem ein Führungsloch 41 vorgesehen ist, durch das ein Verbindungsdraht 42 verläuft, der den distalen Steuerblock 35 sowie den proximalen Steuerblock 36 auf Zug miteinander verbindet.

Zwischen dem Anschlag 39 und dem distalen Steuerblock 36 ist eine freie Wegstrecke 43 angedeutet, um die der Steuerblock 36 und damit der Steuerblock 35 gegenüber der Außenhülle 12 in distale Richtung verfahren werden kann. Weil der distale Steuerblock 35 fest mit dem Stentträger 34 und dieser fest mit der Spitze 18 verbunden ist, bestimmt die lichte Weite 43 damit auch den maximalen Verfahrweg, um den die Spitze 18 gegenüber der Außenhülle 12 in distale Richtung verschoben werden kann.

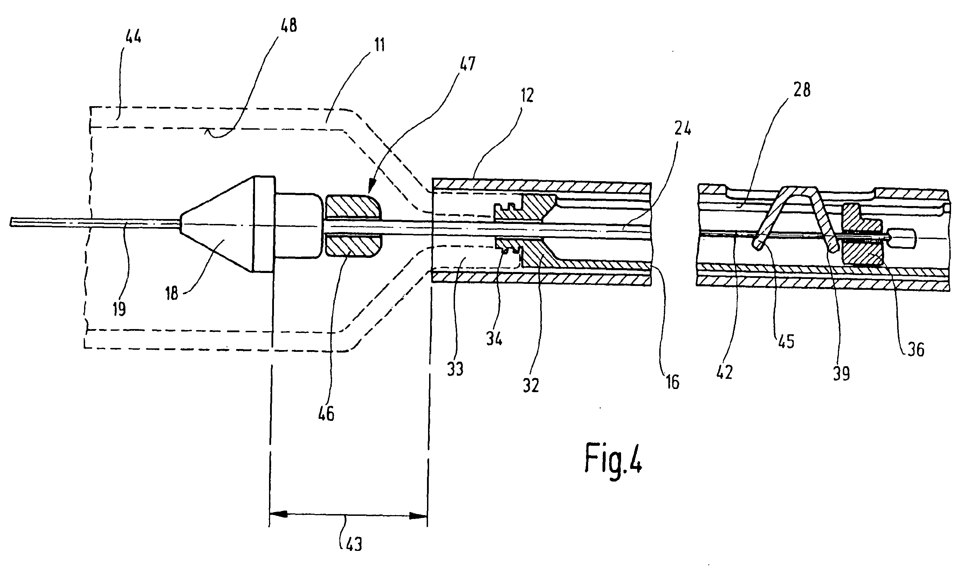

Die Steuerblöcke 35 und 36 bilden zusammen mit dem Verbindungsdraht 42 sowie dem Anschlag 39 damit einen Verriegelungsmechanismus, der die Bewegung des Stentträgers 24 relativ zu der Außenhülle in distaler Richtung auf den maximalen Verfahrweg 43 begrenzt. Beim Herunterschieben des Flechtstents 11 von dem Stentträger 34 bewegt sich damit die Spitze 18 maximal um den Verfahrweg 43 in distale Richtung über die Außenhülle 12 hinaus, wie dies in Fig. 4 schematisch gezeigt ist. Fig. 4 zeigt in einer Darstellung wie Fig. 3 ein weiteres Ausführungsbeispiel des neuen Einführsystems, wobei dort jedoch der Flechtstent 11 bereits teilweise freigesetzt wurde, so dass er mit seinem distalen Ende 44 in distaler Richtung über die Spitze 18 vorsteht. Dies wird erreicht, indem nach dem Positionieren des Einführsystems in dem Gefäß zunächst die Außenhülle 12 um den Verfahrweg 43 zurückgezogen oder aber die Innenhülse 16 gegenüber der Außenhülle 12 in distale Richtung vorgeschoben wird, so dass die Spitze 18 distal um den Verfahrweg 43 vor der Außenhülle 12 befindet. Dann wird durch distales Vorschieben der Innenhülse 16 der Flechtstent 11 allmählich von dem Stentträger 24 heruntergeschoben, so dass sich dieser radial aufweitet und mit seinem distalen Ende 44 über die Spitze 18 in distale Richtung wandert.

Wenn bei der in Fig. 4 gezeigten Situation jetzt die Außenhülle 12 zurückgezogen wird, so weitet sich der Flechtstent 11 in proximale Richtung weiter auf, wobei er sich jedoch nicht stark verkürzen kann, da er sowohl an seinem distalen Ende 44 als auch an seinem proximalen Ende 33 gehalten wird. Um den Flechtstent 11 jetzt sehr dicht aufzubauen, also ihm die Möglichkeit zu geben, sich in axiale Richtung maximal zusammenzuziehen, wird nicht nur die Außenhülle 12 zurückgezogen, gleichzeitig oder stattdessen wird auch die Innenhülse 16 vorgeschoben, so dass sozusagen Stentmaterial aus dem noch gespeicherten Bereich des Flechtstents 11 nachgeliefert wird.

Auf diese Weise ist es möglich, durch relatives Verschieben von Außenhülle 12 und Innenhülse 16 zueinander einen Flechtstent 11 optimal in einem Gefäß abzusetzen und dabei durch entsprechendes Handhaben dafür zu sorgen, dass abwechselnd dicht aufgebaute und weniger dicht aufgebaute Bereiche des Flechtstents 11 so entstehen, wie dies aus medizinischer Sicht wünschenswert ist.

Diese Möglichkeit ergibt sich bei dem Einführsystem 10 dadurch, dass die Spitze 18 gegenüber der Innenhülse 16 frei verschieblich ist. Wenn die Innenhülse 16 in Fig. 4 in distale Richtung geschoben wird, ändert dies die Lage der Spitze 18 nicht. Wird die Außenhülle 12 dagegen in proximale Richtung gezogen, so wird dadurch die Spitze 16 ebenfalls in proximale Richtung zurückgezogen. Auf diese Weise ist es möglich, die Spitze 18 unmittelbar bis an ein Organ oder beispielsweise eine Hirnarterie heranzuführen und beim Freisetzen den Flechtstent 11 sogar noch in distale Richtung weiter über die Spitze 18 zu schieben.

Eine derartige gezielte Positionierung und ein gezieltes Aufbauen des Flechtstents in dem Blutgefäß ist mit bekannten Einführsystemen nicht möglich gewesen.

In Fig. 4 ist rechts noch zu erkennen, dass zusätzlich zu dem ersten Anschlag 39 mit der Außenhülle 12 noch eine Lasche 45 verbunden ist, durch die eine noch bessere Führung des Verbindungsdrahtes 42 im Inneren der Innenhülse 16 möglich ist.

Wenn jetzt der Flechtstent 11 teilweise oder vollständig wieder in die Außenhülle 12 eingezogen werden soll, besteht die Gefahr, dass durch die Reibung zwischen Flechtstent 11 und Stentträger 24 dieser in proximale Richtung bewegt wird, so dass sich der Flechtstent 11 zwischen Außenhülle 12 und Spitze 18 verklemmen kann. Um dies zu verhindern, ist in Fig. 4 gezeigt, dass auf dem Stentträger 24 ein Gleitkörper 46 vorgesehen ist, der bei vollständig geladenem Flechtstent 11 zwischen Spitze 18 und distalem Ende 44 des Flechtstents 11 innen in der Außenhülle 12 sitzt.

Der Gleitkörper 46 ist aus einem elastischen Material gefertigt und an seinem proximalen Ende 47 abgerundet ausgebildet.

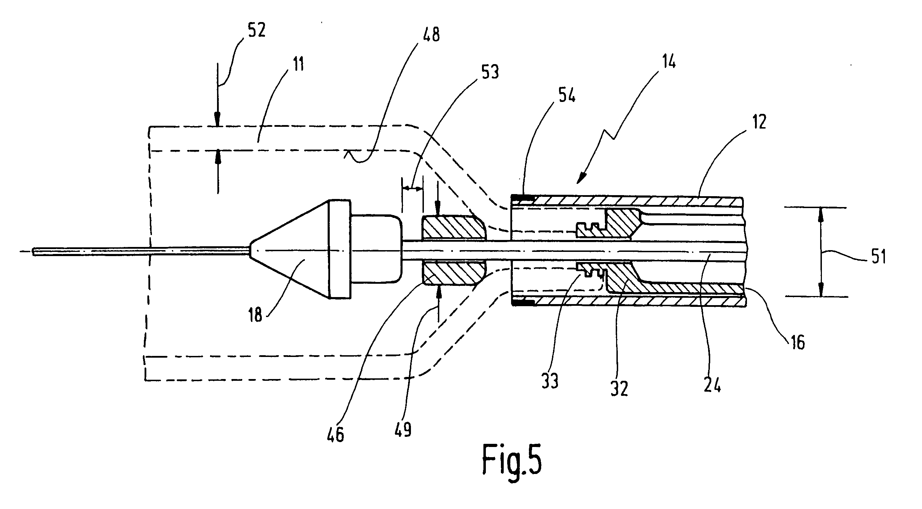

In Fig. 5 ist in einer Darstellung wie Fig. 4 gezeigt, wie der Gleitkörper 46 es verhindert, dass der Flechtstent 11 beim Einziehen in die Außenhülle 12 verklemmt. Zu diesem Zweck weist der Gleitkörper 46 einerseits einen Außendurchmesser 49 auf, der größer ist als die Differenz zwischen dem bei 51 angedeuteten Innendurchmesser der Außenhülle 12 an derem distalen Ende 14 sowie dem Doppelten der bei 52 angedeuteten Wanddicke des Flechtstents 11.

Durch diese Anordnung wird erreicht, dass der Gleitkörper 46 beim Zurückziehen des Flechtstents 11 in proximale Richtung distal weit vor dem distalen Ende 14 der Außenhülle 12 verbleibt, so dass die Gefahr verringert ist, dass der Flechtstent 11 sich verklemmt.

Darüber hinaus ist der Gleitkörper 36 aus einem Material gefertigt, das gegenüber der Innenwand 48 des Flechtstents 11 einen geringen Gleitreibungskoeffizienten aufweist, was ebenfalls dazu führt, dass der Gleitkörper 26 in Fig. 5 nicht in proximale Richtung gezogen wird.

Sollte der Gleitkörper 46 sich dennoch in distale Richtung bewegen, wird er aufgrund seiner Elastizität durch den Flechtstent verformt, wobei sich diese Deformationsenergie periodisch entlädt und den Gleitkörper 46 in distale Richtung springen lässt, was zu einer bei 53 angedeuteten Oszillationsamplitude führt.

Damit sich bei der in Fig. 5 gezeigten Lage des Gleitkörpers 46 das distale Ende 14 der Außenhülle 12 nicht aufweitet, was trotz der Elastizität des Gleitkörpers 46 zu einem Verklemmen führen könnte, ist die Außenhülle 12 an ihrem distalen Ende 14 versteift, sie weist einen Röntgenmarker 54 auf, der zu einer Verstärkung des Materials der Außenhülle 12 führt.

Mit anderen Worten, beim Zurückziehen des Flechtstents 11 in die Außenhülle 12 oszilliert der Gleitkörper 46 zwischen der in Fig. 5 gezeigten Stellung und der in Fig. 4 gezeigten Stellung hin und her und verhindert so ein Verklemmen des Flechtstents 11, der auf diese Weise vollständig wieder in die Außenhülle 12 eingezogen werden kann.

Ein geeignetes Material für den Gleitkörper 46 ist Teflon, die Außenhülle 12 wie der Stentträger 24 sind vorzugsweise aus Thermoplast gefertigt, während die Innenhülse 16 zum Teil aus Edelstahl gefertigt sein kann.

Der Durchmesser 49 beträgt beispielsweise 1,4 mm, der Innendurchmesser 51 liegt bei 1,5 mm und die Wandstärke 52 beträgt 0,2 mm. Der Flechtstent hat im expandierten Zustand dann z.B. eine Länge von 40 mm und einen Außendurchmesser von 6 mm bei einem Flechtwinkel von .α. = 10°.

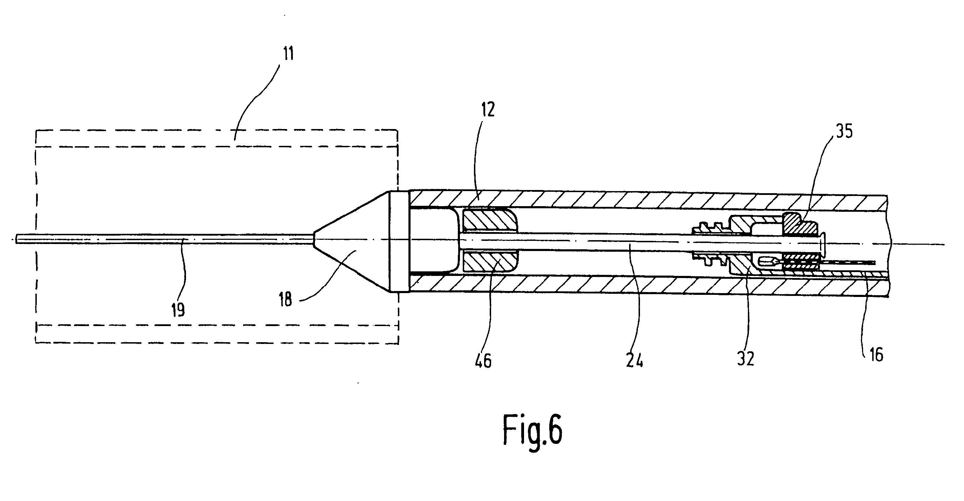

In Fig. 6 ist das Einführsystem 10 aus den Fig. 4 und 5 in einem Zustand gezeigt, bei dem der Flechtstent 11 vollständig freigesetzt wurde. Durch Zurückziehen der Innenhülse 16 gelangt der distale Steuerblock 35 schließlich in Anlage mit dem distalen Ende 32 der Innenhülse 16 und zieht somit über den Stentträger 24 die Spitze 16 wieder in die Außenhülle 12 hinein. In dieser, in Fig. 6 gezeigten Stellung kann das Einführsystem 10 jetzt über den Führungsdraht 19 aus dem Blutgefäß zurückgezogen werden.

In einem konkreten Ausführungsbeispiel kann das Einführsystem aus den Fig. 4 bis 6 eine Außenhülle 12 umfassen, die einen Außendurchmesser von 2,0 mm, einen Innendurchmesser von 1,55 mm und eine Länge von 1550 mm aufweist. Die Außenhülle 12 besteht aus Polytetrafluorethylen (PTFE)/Polyetherblockamid (PEBA), und ist verstärkt oder koextrudiert, z.B. innen aus PTFE und außen aus Polyamid (PA).

Die Innenhülse 16 hat dann beispielsweise einen Außendurchmesser von 1,4 mm, einen Innendurchmesser von 0,4 mm und eine Länge von 200 mm und besteht aus einer Kombination aus Edelstahl (1.4301/1.4310) und einem drucksteifen Material wie Polyetheretherketon (PEEK).

Der Stentträger 24 weist einen Außendurchmesser von 0,6 mm, einen Innendurchmesser von 0,4 mm sowie eine Länge von 200 mm auf und ist aus Polyimid (PI) gefertigt. Der Gleitkörper 46 weist einen Außendurchmesser von 1,5 mm, einen Innendurchmesser von 0,7 mm sowie eine Länge von 2 mm auf, er ist aus PTFE, PEEK oder Polyoxymethylen (POM) gefertigt.

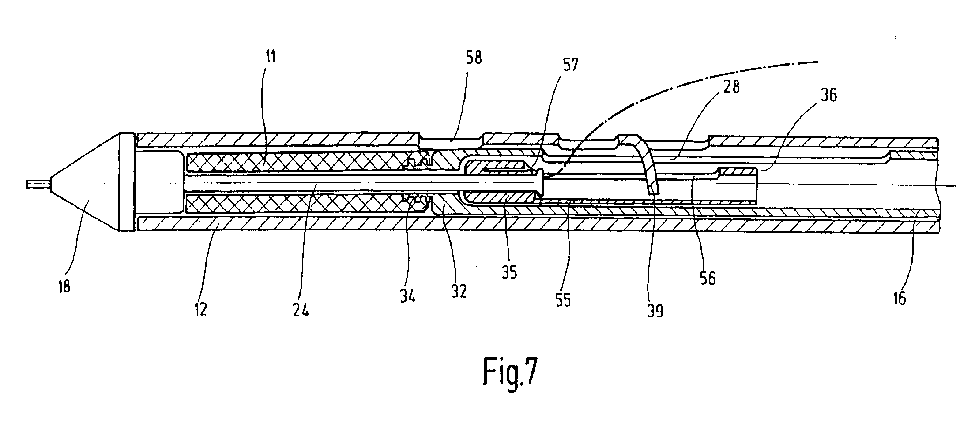

In Fig. 7 ist in einer Darstellung wie Fig. 2 ein weiteres Ausführungsbeispiel des neuen Einführsystems gezeigt.

Während bei dem Ausführungsbeispiel der Fig. 4 bis 6 der Gleitkörper 46 es verhindert, dass der Flechtstent 11 sich beim Zurückziehen in die Außenhülle 12 gegen die Spitze 18 verklemmt, ist bei dem Ausführungsbeispiel der Fig. 7 bis 9 der Gleitkörper 46 nicht erforderlich, der distale Steuerblock 35 und der proximale Steuerblock 36 sind hier an einer Steuerhülse 55 ausgebildet, die verschieblich in der Innenhülse 16 gelagert ist. Die Steuerhülse 55 weist einen dem Längsschlitz 28 zugeordneten Längsschlitz 56 auf, durch den der Anschlag 39 in das Innere der Steuerhülse 55 hineingreift. Auf diese Weise ist wieder der maximale Verfahrweg sichergestellt, um den die Spitze 18 gegenüber der Außenhülle 12 in distale Richtung verfahren werden kann.

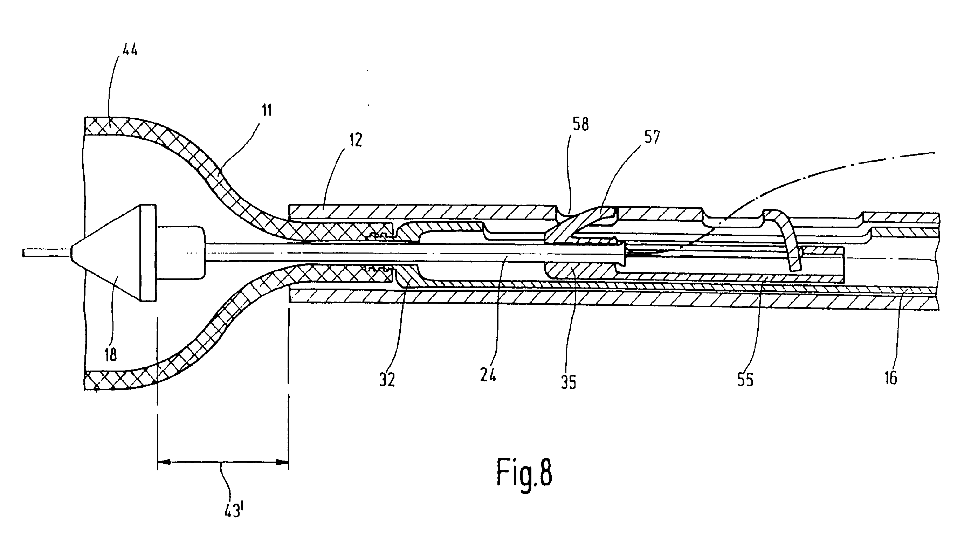

Um jetzt ein unbeabsichtigtes Zurückschieben der Spitze 18 in proximale Richtung zu verhindern, ist an dem distalen Steuerblock 35 ein weiterer Anschlag 57 vorgesehen, der in der in Fig. 7 gezeigten Stellung radial nach innen gedrückt ist und gegen die Innenhülse 16 anliegt. Dieser weitere Anschlag 57 gelangt bei der in Fig. 8 gezeigten Stellung in Eingriff mit einer Ausnehmung 58 in der Außenhülle 12 und verhindert so eine Bewegung des distalen Steuerblockes 35 und damit des Stentträgers 24 sowie der Spitze 18 in proximale Richtung.

Die in Fig. 8 gezeigte Stellung wird eingenommen, sobald die Spitze 18 in distale Richtung um einen vorgegebenen Verfahrweg bewegt wurde, der in Fig. 8 mit 43' bezeichnet ist. Dieser vorgegebene Verfahrweg 43' ist geringfügig kürzer als der maximale Verfahrweg 43, der in Fig. 4 gezeigt ist. Auch wenn jetzt bei der Situation der Fig. 8 die Innenhülse 16 in proximale Richtung bewegt wird, zieht sie den Flechtstent 12 auf den Stentträger 24. Trotz der zwischen Stentträger 24 und Flechtstent 11 herrschenden Reibung wird der Stentträger 24 nicht in proximale Richtung bewegt, dies wird durch den weiteren Anschlag 57 nämlich verhindert.

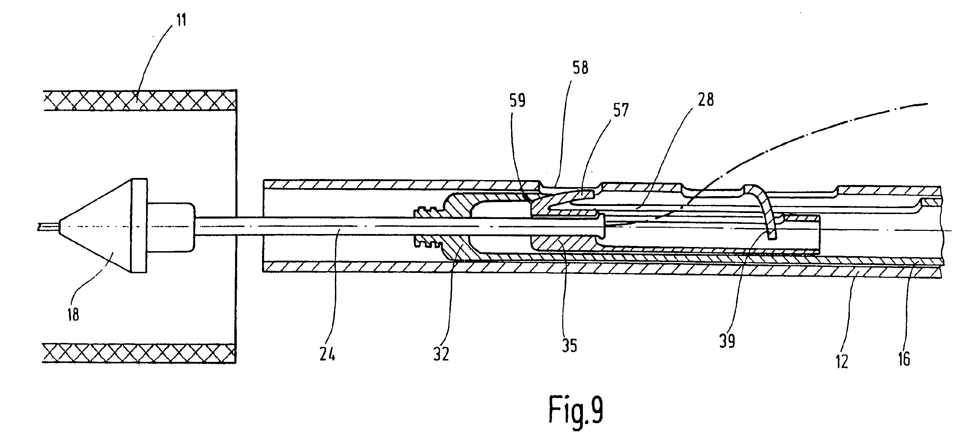

Dennoch ist es möglich, auch die Spitze 18 wieder vollständig in die Außenhülle 12 einzuziehen, weil nämlich beim weiteren proximalen Verfahren der Innenhülse 16 diese schließlich mit dem distalen Ende 59 des Längsschlitzes 28 auf den weiteren Anschlag 57 trifft und diesen radial nach innen drückt, so dass er wieder aus der Ausnehmung 58 freikommt, wie dies in Fig. 9 gezeigt ist, wo der Flechtstent 11 bereits vollständig freigesetzt wurde, so dass die Spitze 18 jetzt vollständig in die Außenhülle 12 zurückgezogen wird, um das Einführsystem aus dem Gefäß entnehmen zu können.

## Patentansprüche

1. Einführsystem mit einem selbstexpandierenden, zur Implantation in ein Blutgefäß vorgesehenen Flechtstent (11),
mit einer ein distales und ein proximales Ende (14, 15) aufweisenden Außenhülle, in der eine relativ zu der Außenhülle (12) verschiebbare Innenhülse (16) angeordnet ist, die an dem proximalen Ende (15) aus der Außenhülle (12) mit einem Handhabungsabschnitt (17) herausragt, und mit einer an dem distalen Ende (14) angeordneten Spitze (18), die fest mit einem ein distales und ein proximales Ende aufweisenden Stentträger (24) verbunden ist, auf dessen distalen Ende der Flechtstent (11) in seinem geladenen Zustand angeordnet ist, wobei der Stentträger (24) relativ zu der Innenhülse (16) verschieblich angeordnet ist und mit seinem proximalen Ende in die Innenhülse (16) hineinragt, wobei die Innenhülse an ihrem distalen Ende (32) eine Halterung aufweist, über die der Flechtstent (11) an seinem proximalen Ende (33) auf Zug mit der Innenhülse (16) verbunden ist,
**dadurch gekennzeichnet, dass** zwischen Außenhülle (12) und Stentträger (24) ein Verriegelungsmechanismus vorgesehen ist, der die Bewegung des Stentträgers (24) relativ zu der Außenhülle (12) in distaler Richtung auf einen maximalen Verfahrweg (43) begrenzt.

2. Einführsystem nach Anspruch 1, **dadurch gekennzeichnet, dass** der Verriegelungsnnechanismus die Bewegung des Stentträgers (24) relativ zu der Außenhülle (12) in proximaler Richtung dann begrenzt, wenn der Stentträger (24) zuvor relativ zu der Außenhülle (12) um einen vorgegebenen Verfahrweg (43') in distaler Richtung verfahren wurde.

3. Einführsystem nach Anspruch 1 oder 2, **dadurch gekennzeichnet, dass** mit der Innenhülse (16) ein Entriegelungsmechanismus verbunden ist, der bei proximaler Bewegung der Innenhülse (16) relativ zu der Außenhülle (12) die relative Bewegung des Stentträgers (24) zu der Außenhülle (12) wieder freigibt.

4. Einführsystem nach einem der Ansprüche 1 bis 3, **dadurch gekennzeichnet, dass** der Stentträger (24) an seinem proximalen Ende (25) mit einer Steuerstange (26) verbunden ist, die die Innenhülse (16) zentrisch durchsetzt und an dem proximalen Ende (15) aus der Innenhülse (16) herausragt.

5. Einführsystem nach einem der Ansprüche 1 bis 3, **dadurch gekennzeichnet, dass** der Stentträger (24) an seinem proximalen Ende (25) in der Innenhülse (16) gelagert ist, die einen Längsschlitz (28) aufweist, durch den ein mit der Außenhülle (12) verbundener erster Anschlag (39) in die Innenhülse (16) hineinragt, der die Bewegung des Stentträgers (24) relativ zu der Außenhülle (12) in distaler Richtung auf dem maximalen Verfahrweg (43) begrenzt.

6. Einführsystem nach Anspruch 5, **dadurch gekennzeichnet, dass** der Stentträger (24) fest mit einem verschieblich in der Innenhülse (16) gelagerten distalen Steuerblock (35) verbunden ist, der auf Zug mit einem verschieblich in der Innenhülse (16) gelagerten proximalen Steuerblock (36) verbunden ist, der bei relativer Bewegung des Stentträgers (24) zu der Außenhülle (12) in distaler Richtung um den maximalen Verfahrweg (43) mit dem ersten Anschlag (39) in Anlage gelangt.

7. Einführsystem nach Anspruch 5 oder 6, **dadurch gekennzeichnet, dass** dem Längsschlitz (28) ein mit dem Stentträger (24) verbundener weiterer Anschlag (57) zugeordnet ist, der in Anlage mit einer Ausnehmung (58) an der Außenhülle (12) gelangt und die Bewegung des Stentträgers (24) relativ zu der Außenhülle (12) in proximaler Richtung begrenzt, wenn der Stentträger (24) zuvor relativ zu der Außenhülle (12) um den vorgegebenen Verfahrweg (43') in distaler Richtung verfahren wurde.

8. Einführsystem nach den Ansprüchen 6 und 7, **dadurch gekennzeichnet, dass** der weitere Anschlag (57) an dem distalen Steuerblock (35) angeordnet ist.

9. Einführsystem nach einem der Ansprüche 6 bis 8, **dadurch gekennzeichnet, dass** der distale und der proximale Steuerblock (35, 36) über einen Verbindungsdraht (42) miteinander verbunden sind, der vorzugsweise durch ein in dem ersten Anschlag (39) vorgesehenes Führungsloch (41) verläuft.

10. Einführsystem nach einem der Ansprüche 6 bis 8, **dadurch gekennzeichnet, dass** der distale und der proximale Steuerblock (35, 36) an gegenüberliegenden Enden einer in der Innenhülse (16) verschieblich gelagerten Steuerhülse (55) ausgebildet sind, in der ein Längsschlitz (46) vorgesehen ist, in den der erste Anschlag (39) eingreift.

11. Einführsystem nach einem der Ansprüche 1, 5, 6, 9 oder 10, **dadurch gekennzeichnet, dass** auf den Stentträger (24) ein längs verschiebbarer Gleitkörper (46) angeordnet ist, der bei geladenem Flechtstent (11) zwischen Flechtstent (11) und Spitze (18) liegt.

12. Einführsystem nach Anspruch 11, **dadurch gekennzeichnet, dass** der Gleitkörper (46) einen Außendurchmesser (49) aufweist, der größer ist als die Differenz zwischen dem Innendurchmesser (51) der Außenhülle (12) an derem distalen Ende (14) und der doppelten Wandstärke (52) des Flechtstents (11).

13. Einführsystem nach Anspruch 11 oder 12, **dadurch gekennzeichnet, dass** der Gleitkörper (46) an seinem proximalen Ende (47) abgerundet ist.

14. Einführsystem nach einem der Ansprüche 11 bis 13, **dadurch gekennzeichnet, dass** der Gleitkörper (46) aus einem Material gefertigt ist, das gegenüber der Innenwand (48) des Flechtstents (11) einen geringen Gleitreibungskoeffizienten aufweist.

15. Einführsystem nach einem der Ansprüche 11 bis 14, **dadurch gekennzeichnet, dass** der Gleitkörper (46) aus einem elastischen Material gefertigt ist.

16. Einführsystem nach einem der Ansprüche 11 bis 15 **dadurch gekennzeichnet, dass** das distale Ende (14) der Außenhülle (12) versteift ist.

17. Einführsystem nach einem der Ansprüche 1 bis 16, **dadurch gekennzeichnet, dass** auf dem Handhabungsabschnitt (17) der Innenhülse (16) ein Distanzstück (23) fixiert ist, das ein Einschieben der Innenhülse (16) in die Außenhülle (12 begrenzt.

18. Einführsystem nach einem der Ansprüche 1 bis 17, **dadurch gekennzeichnet, dass** die Halterung der Innenhülse (16) einen Flansch (34) aufweist, auf dem das proximale Ende (33) des Flechtstents (11) durch die Außenhülle (12) fixiert wird.

19. Einführsystem nach einem der Ansprüche 1 bis 18, **dadurch gekennzeichnet, dass** zentral durch die Spitze (18) und den Stentträger (24) ein Kanal (27) für einen Führungsdraht (19) verläuft, der seitlich durch Längsschlitze (29, 31) aus der Innenhülse (16) und der Außenhülle (12) herausgeführt ist.

## Claims

1. A delivery system with a self-expanding braided stent (11) intended for implantation into a blood vessel, having
an outer sleeve, which has a distal end (14) and a proximal end (15) and in which an inner sleeve (16) is arranged which is displaceable relative to the outer sleeve (12) and protrudes, with a handling portion (17), from the proximal end (15) of the outer sleeve (12), and with a tip (18), which is arranged at the distal end (14) and is securely connected to a stent support (24) having a proximal and a distal end, on which distal end the braided stent (11) is arranged in its loaded state, wherein the stent support (24) is arranged to be displaceable relative to the inner sleeve (16) and protrudes with its proximal end into the inner sleeve (16), wherein the inner sleeve (16) has a holder at its distal end (32), via which holder the proximal end (33) of the braided stent (11) is connected under tension to the inner sleeve (16),
**characterized in that** between outer sleeve (12) and stent support (24), a locking mechanism is provided by which the movement of the stent support (24) relative to the outer sleeve (12) in the distal direction is limited to a maximum path of travel (43).

2. The delivery system as claimed in claim 1, **characterized in that** the locking mechanism limits the movement of the stent support (24) relative to the outer sleeve (12) in the proximal direction when the stent support (24) has previously been moved relative to the outer sleeve (12) in the distal direction by a predetermined path of travel (43').

3. The delivery system as claimed in claim 1 or 2, **characterized in that** an unlocking mechanism is connected to the inner sleeve (16) and, upon proximal movement of the inner sleeve (16) relative to the outer sleeve (12), again frees the movement of the stent support (24) relative to the outer sleeve (12).

4. The delivery system as claimed in one of claims 1 to 3, **characterized in that** the stent support (24) is connected at its proximal end (25) to a control rod (26), which extends centrally through the inner sleeve (16) and protrudes at the proximal end (15) from the inner sleeve (16).

5. The delivery system as claimed in one of claims 1 to 3, **characterized in that** the stent support (24) is at its proximal end (25) in the inner sleeve (16), which has a longitudinal slit (28) through which a first limit stop (39) connected to the outer sleeve (12) protrudes into the inner sleeve (16), by means of which limit stop (39) the movement of the stent support (24) relative to the outer sleeve (12) in the distal direction is limited to the maximum path of travel (43).

6. The delivery system as claimed in claim 5, **characterized in that** the stent support (24) is securely connected to a distal control block (35), which is mounted displaceably in the inner sleeve (16) and which is connected under tension to a proximal control block (36) mounted displaceably in the inner sleeve (16), which proximal control block (36) comes into contact with the first limit stop (39) upon movement of the stent support (24) relative to the outer sleeve (12) in the distal direction by the maximum path of travel (43).

7. The delivery system as claimed in claim 5 or 6, **characterized in that** the longitudinal slit (28) is assigned a further limit stop (57), which is connected to the stent support (24) and which comes into contact with a recess (58) on the outer sleeve (12) and limits the movement of the stent support (24) relative to the outer sleeve (12) in the proximal direction when the stent support (24) has previously been moved relative to the outer sleeve (12) by the predetermined path of travel (43') in the distal direction.

8. The delivery system as claimed in claims 6 and 7, **characterized in that** the further limit stop (57) is arranged on the distal control block (35).

9. The delivery system as claimed in one of claims 6 to 8, **characterized in that** the distal and proximal control blocks (35, 36) are connected to each other by a connecting wire (42), which preferably extends through a guide hole (41) provided in the first limit stop (39).

10. The delivery system as claimed in one of claims 6 to 8, **characterized in that** the distal and proximal control blocks (35, 36) are formed at opposite ends of a control sleeve (55), which is mounted displaceably in the inner sleeve (16) and in which a longitudinal slit (46) is provided into which the first limit stop (39) engages.

11. The delivery system as claimed in one of claims 1, 5, 6, 9 or 10, **characterized in that** a longitudinally displaceable slide body (46) is arranged on the stent support (24) and lies between the braided stent (11) and the tip (18) when the braided stent (11) is in the loaded state.

12. The delivery system as claimed in claim 11, **characterized in that** the slide body (46) has an external diameter (49) that is greater than the difference between the internal diameter (51) of the outer sleeve (12) at its distal end (14) and twice the wall thickness (52) of the braided stent (11).

13. The delivery system as claimed in claim 11 or 12, **characterized in that** the slide body (46) is rounded at its proximal end (47).

14. The delivery system as claimed in one of claims 11 to 13, **characterized in that** the slide body (46) is made of a material that has a low coefficient of kinetic friction relative to the inner wall (48) of the braided stent (11).

15. The delivery system as claimed in one of claims 11 to 14, **characterized in that** the slide body (46) is made of an elastic material.

16. The delivery system as claimed in one of claims 11 to 15, **characterized in that** the distal end (14) of the outer sleeve (12) is stiffened.

17. The delivery system as claimed in one of claims 1 to 16, **characterized in that** a spacer (23) is fixed on the handling portion (17) of the inner sleeve (16) and limits the sliding of the inner sleeve (16) into the outer sleeve (12).

18. The delivery system as claimed in one of claims 1 to 17, **characterized in that** the holder has a flange (34) on which the proximal end (33) of the braided stent (11) is fixed by the outer sleeve (12).

19. The delivery system as claimed in one of claims 1 to 18, **characterized in that** a channel (27) for a guide wire (19) extends centrally through the tip (18) and the stent support (24), which guide wire (19) is guided laterally out of the inner sleeve (16) and outer sleeve (12) through longitudinal slits (29, 31).

## Revendications

1. Système d'insertion avec un stent tressé auto-expansible (11) prévu pour l'implantation dans un vaisseau sanguin,
ayant une enveloppe extérieure présentant une extrémité distale et une extrémité proximale (14, 15), dans laquelle est disposée une douille intérieure (16) déplaçable par rapport à l'enveloppe extérieure (12), qui fait saillie hors de l'enveloppe extérieure (12) à son extrémité proximale (15) avec une section de maniement (17), et ayant une pointe (18) disposée à son extrémité distale (14), qui est reliée fermement à un support de stent (24) présentant une extrémité proximale et une extrémité distale, extrémité distale sur laquelle, à son état chargé, le stent tressé (11) est disposé, sachant que le support de stent (24) peut être disposé d'une manière déplaçable par rapport à la douille intérieure (16) et s'enfonce avec son extrémité proximale dans la douille intérieure (16), que le douille intérieure présente à son extrémité distale (32) une attache de maintien, grâce à laquelle le stent tressé (11) est relié en traction par son extrémité proximale (33) à la douille intérieure (16),
**caractérisé en ce qu'**il est prévu, entre l'enveloppe extérieure (12) et le support de stent (24), un mécanisme de verrouillage, qui limite le mouvement du support de stent (24) par rapport à l'enveloppe extérieure (12) en direction distale à un trajet de déplacement maximal (43).

2. Système d'insertion selon la revendication 1, **caractérisé en ce que** le mécanisme de verrouillage limite le mouvement du support de stent (24) par rapport à l'enveloppe extérieure (12) en direction proximale dans le cas où le support de stent (24) a d'abord été déplacé auparavant en direction distale par rapport à l'enveloppe extérieure (12) d'un trajet de déplacement prédéterminé (43').

3. Système d'insertion selon la revendication 1 ou 2, **caractérisé en ce qu'**un mécanisme de déverrouillage est relié à la douille intérieure (16), lequel mécanisme, en cas de mouvement proximal de la douille intérieure (16) par rapport à l'enveloppe extérieure (12), libère à nouveau le mouvement relatif du support de stent (24) par rapport à l'enveloppe extérieure (12).

4. Système d'insertion selon l'une quelconque des revendications 1 à 3, **caractérisé en ce que** le support de stent (24) est relié par son extrémité proximale (25) à une tige de commande (26), qui traverse de manière centrée la douille intérieure (16) et qui fait saillie à l'extrémité proximale (15) hors de la douille intérieure (16).

5. Système d'insertion selon l'une quelconque des revendications 1 à 3, **caractérisé en ce que** le support de stent (24) est logé à son extrémité proximale (25) dans la douille intérieure (16), qui présente une gorge longitudinale (28), à travers laquelle s'enfonce, dans la douille intérieure (16), une première butée (39) reliée à l'enveloppe extérieure (12), qui limite le mouvement du support de stent (24) par rapport à l'enveloppe extérieure (12) en direction distale au trajet de déplacement maximal (43).

6. Système d'insertion selon la revendication 5, **caractérisé en ce que** le support de stent (24) est relié de manière ferme à un bloc de commande distal (35) logé de manière décalable dans la douille intérieure (16), lequel bloc est relié en traction à un bloc de commande proximal (36) logé de manière décalable dans la douille intérieure (16), qui vient à s'appuyer sur la première butée (39), en cas de mouvement relatif du support de stent (24) par rapport à l'enveloppe extérieure (12) en direction distale à raison du trajet de déplacement maximal (43).

7. Système d'insertion selon la revendication 5 ou 6, **caractérisé en ce que** l'on affecte à la gorge longitudinale (28) une butée supplémentaire (57) reliée au support de stent (24), laquelle butée vient à s'appuyer sur un évidement (58) à l'enveloppe extérieure (12) et limite le mouvement du support de stent (24) par rapport à l'enveloppe extérieure (12) en direction proximale, lorsque le support de stent (24) a été déplacé auparavant par rapport à l'enveloppe extérieure (12) d'un trajet de déplacement prédéterminé (43) en direction distale.

8. Système d'insertion selon les revendications 6 et 7, **caractérisé en ce que** la butée supplémentaire (57) est disposée sur le bloc de commande distal (35).

9. Système d'insertion selon l'une quelconque des revendications 6 à 8, **caractérisé en ce que** les blocs de commande distal et proximal (35, 36) sont reliés l'un à l'autre à l'aide d'un fil de raccordement (42), qui se déplace de préférence à travers un trou de guidage (41) prévu dans la première butée (39).

10. Système d'insertion selon l'une quelconque des revendications à 6 à 8, **caractérisé en ce que** les blocs de commande distal et proximal (35, 36) sont formés aux extrémités opposées d'une douille de commande (55) logée de manière déplaçable dans la douille intérieure (16), douille de commande dans laquelle est prévue une gorge longitudinale (46), dans laquelle la première butée (39) s'engrène.

11. Système d'insertion selon l'une quelconque des revendications 1, 5, 6, 9 ou 10, **caractérisé en ce que** l'on dispose sur le support de stent (24) une pièce coulissante (46) décalable longitudinalement, qui est située, en cas de stent tressé (11) chargé, entre le stent tressé (11) et la pointe (18).

12. Système d'insertion selon la revendication 11, **caractérisé en ce que** la pièce coulissante (46) présente un diamètre extérieur (49), qui est plus grand que la différence entre le diamètre intérieur (51) de l'enveloppe extérieure (12) à son extrémité distale (14) et le double de l'épaisseur de paroi (52) du stent tressé (11).

13. Système d'insertion selon la revendication 11 ou 12, **caractérisé en ce que** la pièce coulissante (46) est de forme arrondie à son extrémité proximale (47).

14. Système d'insertion selon l'une quelconque des revendications 11 à 13, **caractérisé en ce que** la pièce coulissante (46) est fabriquée à l'aide d'un matériau qui présente, par rapport à la paroi intérieure (48) du stent tressé (11), un coefficient de friction de glissement plus faible.

15. Système d'insertion selon l'une quelconque des revendications 11 à 14, **caractérisé en ce que** la pièce coulissante (46) est fabriquée à l'aide d'un matériau élastique.

16. Système d'insertion selon l'une quelconque des revendications 11 à 15, **caractérisé en ce que** l'extrémité distale (14) de l'enveloppe extérieure (12) est renforcée.

17. Système d'insertion selon l'une quelconque des revendications 1 à 16, **caractérisé en ce que** l'on fixe sur la section de maniement (17) de la douille intérieure (16) une pièce d'écartement (23) qui limite l'introduction de la douille intérieure (16) dans l'enveloppe extérieure (12).

18. Système d'insertion selon l'une quelconque des revendications 1 à 17, **caractérisé en ce que** la fixation de la douille intérieure (16) présente une bride (34) sur laquelle est fixée à travers l'enveloppe extérieure (12) l'extrémité proximale (33) du stent tressé (11).

19. Système d'insertion selon l'une quelconque des revendications 1 à 18, **caractérisé en ce qu'**à travers la pointe (18) et le support de stent (24) passe de manière centrale un canal (27) pour un fil de guidage (19), qui émerge latéralement par des fentes longitudinales (29, 31) hors de la douille intérieure (16) et de l'enveloppe extérieure (12).
